# EUROPEAN PATENT SPECIFICATION

(11) **EP 0 380 940 B1**
(45) Date of publication and mention of the grant of the patent: **05.10.1994**
(21) Application number: 90100544.7
(22) Date of filing: 11.01.1990
(51) Int. Cl.: A61K 7/42

(54) **Aqueous skin tanning composition**
Wässriges Hautbräunungsmittel
Composition aqueuse pour le tannage de la peau

(30) Priority: 11.01.1989 NL 8900059
(43) Date of publication of application: 08.08.1990
(73) Proprietor: Chemisch Adviesbureau Drs. J.C.P. Schreuder B.V., NL-3740 AK Baarn (NL)
(72) Inventor: Schreuder, Johannes, Carolus, Petrus, Drs., NL-3740 AK Baarn (NL)
(74) Representative: DIEHL GLAESER HILTL & PARTNER

(56) References cited:
- EP-A- 0 255 964
- FR-A- 2 608 424

## Description

The invention is relating to the use of a composition as a skin tanning composition and more particularly to an aqueous composition, which may effect a relatively fast skin tanning after exposure to natural sunlight or exposure to the light from sun-tanning equipment such as sunbeds or solaria, whereas the skin is not exposed to compounds, which may disturb the biological equilibrium of the skin (so-called hypoallergenic compositions).

In the course of years many compositions for skin tanning already have been proposed. Part of these proposed compositions contain as active main ingredient panthenol, which is applied in an acidic non-aqueous phase. Such compositions are indeed effecting the desired skin tanning after exposure to natural or artificial sunlight, however, according to the general conception, too slowly.

Partly due to this reason compounds, which have to protect the skin temporarily against the excessive effect of harmful frequencies in the light spectrum leading to burning of the skin, before the actual skin tanning process due to the natural production of melanoprotein (pigment) has taken place in a sufficient degree, have to be incorporated in these skin tanning agents.

These so-called light filtering agents are on the other hand still regarded as undesired, due to a possible disturbance of the biological equilibrium of the skin and the toxicity of these compounds for humans in large concentrations, due to accumulation.

From EP-A-0 255 964 an intermediate aqueous composition is known, which comprises as active ingredients panthenol, and preferably D-panthenol, and tyrosine or a compound from which tyrosine easily can be formed in situ, such as esters of tyrosine, which are sufficiently dissolvable in an aqueous phase, or phenyl alanine which may be converted in situ into tyrosine, and which moreover comprises a pH regulating agent.

However, these active ingredients are incorporated in an aqueous phase, which is included in a water-in-oil emulsion, the oily phase of which is composed of an oil fraction, consisting of optionally branched paraffinic oils, esters derived from optionally unsaturated higher natural fatty acids and from higher natural aliphatic alcohols, an emulsifier consisting of mono- and/or diglycerides of higher optionally unsaturated natural fatty acids and ethoxylated glycerides, esterified with optionally unsaturated fatty acids, a stabilizing agent, consisting of modified montmorillonites and a preservative.

In order to obtain an optimal skin tanning additionally one or more secondary components may be added such as vitamin E, glycerine and a gel-forming agent such as carraghenate, and if desired additionally perfume, antioxidants and an alkanol and preferably ethanol. Although such a skin tanning composition effects the desired skin tanning, an increasing demand has developed for a skin tanning composition which can be easily and quickly applied and preferably by spraying. More particularly, an increasing demand has developed for non-irritating, easily applicable and ecologically harmless skin tanning compositions, and more particularly skin tanning spray compositions.

Other skin tanning compositions which were proposed in the past and which were containing panthenol, optionally in combination with synthetic active ingredients, which may effect a skin tanning without active exposure to sunlight or artificial skin tanning light, in fact always consisted of emulsions of an oily or fatty phase and an aqueous phase, such as e.g. disclosed in the published German Patent Application DE-A-2 722 725.

On the other hand, from CH-642 537 were also skin tanning compositions known, which contained as one of the active ingredients tyrosine and/or tyrosine esters in addition to synthetic activators in the form of 3,6-diamino acridine and/or 6,7-dimethyl-9-ribityl-isoalloxazine and an UV-filter.

The skin tanning compositions disclosed therein mainly consist of creams, whereas the spraying composition described in example 1 consists of an alcoholic mixture of the hereinbefore mentioned ingredients, fats (e.g. isopropyl myristate) and emulsifiers and/or surfactants and/or gelling agents and/or thickening agents.

It will be appreciated that according to the generally existing conception under persons skilled in the art, tyrosine as such is very bad to dissolve in an aqueous phase and the usual applied esters of tyrosine in aqueous systems have to be regarded as relatively unstable. Therefore said skilled persons were expecting problems when applying tyrosine or esters thereof in predominantly aqueous systems, which should not contain significant amounts of alcohol and which are not incorporated into water-in-oil/fat or oil/fat-in-water emulsions.

That such a person skilled in the art, being in charge of the development of a cosmetic formulation and more particularly a skin tanning composition, did not primarily consider the use of aqueous suspensions or emulsions on their own during many years, appears e.g. from FR-B-2 326 914, which discloses complex emulsion systems consisting of water-in-oil in water emulsions, prepared from a disperse phase and a dispersion medium, whereby the disperse phase comprises a water-in-oil emulsion and the oily phase is obtained by dissolving an emulsifier into the oil at such a hydrophilic/hydrophobic balance that a water in oil emulsion is formed within the oil in the disperse medium. Said emulsions serve the preparation of cosmetic and pharmaceutical products and foods.

More particularly, in lines 18 - 24 of page 2 is indicated that these complex emulsions provide the advantages of oil-in-water and water-in-oil emulsions in cosmetics and that the products therefore show a strongly cleansing and soothing capability, can be easily spread out and give a refreshing feeling after the application.

As an example of such a composition a sun lotion is described in example 18 on page 33.

The document FR-B-2 608 424 discloses a natural complex which might facilitate skin tanning via promoting the melanogenesis, i.e. the development of the melanine in the skin, and which does not contain any sun-filtering or sun-protecting agent.

Such a complex actually comprises at least nicotinic amide and glutamic acid in combination with L-tyrosine and/or L-phenylalanine and/or several other ingredients, occurring in the human skin tissue, such as L-tryptophane, L-histidine, L-cystine and L-arginine.

However, according to page 2, lines 1 - 8 and claim 11, the complex in powder form has to be composed of a relatively large number of natural, rather expensive ingredients. Although there has been indicated in said French patent that the skin tanning powder complex may be incorporated inter alia in diluted aqueous solutions, by application of the hydrochloric acid addition salts or other water-soluble derivatives, there has not been indicated at all how said diluted stable aqueous solutions may be obtained. I.e. not any specification of specific concentrations of the complex in aqueous compositions and other specific measures are provided, which might help a person skilled in the art to overcome the hereinbefore mentioned problem of the very bad solubility of e.g. tyrosine as such in an aqueous phase and of the relative instability of the usually applied derivatives such as esters of tyrosine in aqueous systems. Therefore said French patent did certainly not make it obvious to persons skilled in the art to compose predominantly aqueous skin tanning compositions which should contain tyrosine or derivatives thereof as possible ingredient.

Surprisingly there has been found now as result of research and experimentation that a composition free of oil and/or fat, except in form of a perfume optionally added, can be applied for the fast and efficient skin tanning aimed at.

Such a composition has been found to comprise only part of the hereinbefore mentioned ingredients, being earlier considered as essential and which are moreover substantially free of alkanol.

Moreover, it has been found that the fast and efficient skin tanning aimed at can even be reached by significantly lower concentrations of these ingredients as compared with those according to the hereinbefore discussed EP-A-0 255 964, and that such compositions cause a refreshing effect.

Accordingly, the invention is relating to the use of a composition which is free of fat and/or oil, except in form of a perfume optionally added, and which is composed of at least the following ingredients:
- panthenol and preferably D-panthenol in an amount of 0.1 - 5.0 % by weight and preferably of 0.1 - 1.0 % by weight and more preferably of 0.4 - 0.8 % by weight, calculated relative to the weight of the complete composition,
- tyrosine or a compound from which tyrosine can be formed in situ, in an amount of 0.1 - 5.0 % by weight, preferably of 0.1 - 1.0 % by weight and more preferably of 0.2 - 0.6 % by weight, calculated as tyrosine relative to the weight of the complete composition,
- a pH adjusting, buffering agent to maintain the pH of the total aqueous composition in the range of from 4 - 7 and preferably in the range of from 4 - 6,
- water ad 100 % by weight,
as a skin tanning agent.

The invention is relating also to a skin tanning composition, characterized in that it is composed of the following combination of ingredients:
- panthenol in an amount of from 0.1 to 5 % by weight, calculated on the weight of the complete composition,
- tyrosine or a compound from which tyrosine may be formed in situ, in an amount of from 0.1 to 5 % by weight, calculated as tyrosine relative to the weight of the complete composition,
- a pH adjusting, buffering agent to keep the pH of the complete aqueous composition in the range of from 4 to 7, and
- water ad 100 % by weight.

As tyrosine derivative, from which tyrosine may easily be formed in situ, may e.g. be applied a water-soluble ester of tyrosine or phenylalanine of which is known that it can be easily converted into tyrosine. Another group of preferred derivatives is formed by esters of tyrosine, such as the methyl, ethyl, (iso)butyl ester or benzyl ester, from which the ethyl ester is more preferred. In such tyrosine derivatives the free amino group and/or free hydroxy group may have been protected, if desired. E.g. the amino group can be protected herein by means of HCl addition or by acylation with a suitable organic acid derivative such as an acid halide. This protection may have been effected also by methylsilanol acetyl tyrosine.

As pH adjusting, buffering agent a great variety of combinations of naturally occurring organic acids, such as citric acid, lactic acid, succinic acid or tartaric acid, and of alkali metal or alkaline earth metal hydroxides and/or alkali metal or alkaline earth metal salts of these acids, may be applied, depending on the specific type and amounts of the main ingredients.

Preferably a combination of lactic acid, succinic acid or citric acid and sodium hydroxide or potassium hydroxide is applied.

The applied amount of the buffering agent will be in the range of from 0.1 - 3 % by weight, calculated relative to the weight of the complete aqueous composition.

Preferably the amount of the buffering agent is from 0.1 - 1 % by weight.

The molar ratio between tyrosine and panthenol preferably is in the range of from 2 : 1 to 1 : 4 and more preferably from 1 : 1 to 1 : 3.

In addition to the hereinbefore mentioned ingredients of the aqueous skin tanning composition, one or more secondary ingredients may be present if desired, such as a preservative and/or perfume.

Different types of preservatives may be applied such as esters of parahydroxy benzoic acid. Known preservatives which may successfully be applied are e.g. Phenonip, Germall or Hydroconserv (registered trademarks) in an amount of from 0.05 - 0.5 % by weight and preferably from 0.1 - 0.3 % by weight, calculated relative to the weight of the complete aqueous composition.

Perfumes of a great variety of types may be applied in an amount of from 0.05 - 0.5 % by weight and preferably of from 0.1 - 0.2 % by weight, calculated relative to the weight of the complete aqueous composition.

It will be appreciated that the aqueous skin tanning compositions as used according to the present invention actually do contain relatively few essential ingredients and preferably in surprisingly small amounts, enabling an easy and cheap production of these aqueous compositions. Moreover said compositions do not contain alkanols in comparison to a number of compositions according to the hereinbefore described publications.

The aqueous skin tanning compositions as used according to the present invention may be applied in the form of a spray, obtained by means of a hand-pump in combination with a spray nozzle, suitable for this purpose, or by means of a suitable propellant in combination with a suitable spray nozzle. As suitable propellants may e.g. be used nitrogen, carbon dioxide, Freon (registered trademark) and dimethoxy methane etc., of which carbon dioxide and dimethoxy methane are preferred. It will be appreciated therefore that the invention is also relating to the use of spray compositions, mainly comprising an aqueous skin tanning composition as hereinbefore specified and an amount of propellant which is suitable for this type of spray compositions.

More particularly the invention is also relating to the use of spray compositions which contain the aqueous composition according to the invention, mixed with e.g. of from 1 - 6 % by weight of carbon dioxide, calculated relative to the weight of the complete mixture, and to the use of spray compositions which contain the aqueous composition according to the invention mixed with e.g. of from 20 - 40 % by weight of dimethoxy methane, calculated relative to the weight of the complete mixture.

It will be appreciated that to the hereinbefore specified skin tanning compositions one or more additional auxiliaries or adjuvants may be added (so-called secondary ingredients) in order to obtain a composition showing the most optimal, preferred characteristics, such as lotions, milks, gels or creams etc.

The aqueous skin tanning compositions as used according to the present invention may be prepared by a process, usual for this purpose, comprising the subsequent addition of a pH adjusting buffering agent, panthenol, tyrosine or compounds from which tyrosine may be formed in situ, to the selected starting amount of water.

To the obtained aqueous mixture preservative and/or perfume may optionally be added.

The temperature during this mixing and stirring operation is at most 30°C and preferably in the range of from 5 - 20°C.

It will be appreciated by a person skilled in the art that the application of the hereinbefore specified aqueous skin tanning compositions, i.e. the treatment of the skin with said compositions, consists of a method being usual for such aqueous compositions, comprising the application of 2 - 20 ml of aqueous skin tanning composition per m² skin surface.

The invention is further illustrated on basis of the subsequent examples, however without restricting its scope to these embodiments.

### Example 1

To 490 g of water the following ingredients are added under stirring:

| | |
|---|---|
| citric acid | 1.25 g |
| potassium hydroxide giving rise to a pH value of 5 | 0.8 g |
| ethyl ester of tyrosine·HCl | 2.0 g |
| d-panthenol | 4.0 g |
| preservative (Germall II) | 1.0 g |
| perfume | 0.5 g |

100 g of the obtained aqueous skin tanning composition was filled in spray bottles provided with a hand-pump, which allows an efficient spraying on the skin.

### Example 2

In the same way as described in Example 1 an aqueous skin tanning composition was composed using:

| | |
|---|---|
| water | 490 g |
| citric acid | 1.25 g |
| sodium hydroxide | 0.8 g |
| phenyl alanine | 2.5 g |
| d-panthenol | 3.0 g |
| preservative (Phenonip) | 2.0 g |

125 ml spray tins, provided with 2 - 3 g carbon dioxide, were filled in the usual way with 97 to 98 g portions of the obtained aqueous composition.

### Example 3

In the same way as in Example 1, an aqueous skin tanning composition was composed from:

| | |
|---|---|
| water | 495 g |
| lactic acid | 1.1 g |
| sodium hydroxide | 1.0 g |
| phenyl alanine | 4.0 g |
| d-panthenol | 3.0 g |
| perfume | 0.5 g |
| preservative (Phenonip) | 1.0 g |

100 g portions of the obtained aqueous composition were filled in the usual way in spray bottles, provided with a hand-pump, allowing an efficient spraying of the composition on the skin.

### Example 4

In the same way as described in Example 1, an aqueous skin tanning composition was composed from:

| | |
|---|---|
| water | 500 g |
| lactic acid | 1.25 g |
| potassium hydroxide | 0.8 g |
| ethyl ester of tyrosine·HCl | 3.0 g |
| d-panthenol | 3.5 g |
| preservative | 1.0 g |
| perfume | 0.5 g |

Spray tins were filled in the usual way with 70 g portions of the obtained composition together with 30 g of dimethoxy methane.

### Example 5

In the same way as described in Example 1, an aqueous skin tanning composition was composed from:

| | |
|---|---|
| water | 500 g |
| succinic acid | 2.5 g |
| sodium hydroxide | 1.0 g |
| ethyl ester of L-tyrosine·HCl | 15.0 g |
| d-panthenol | 20.0 g |
| perfume | 2.0 g |
| preservative (Phenonip) | 3.0 g |

100 g portions of the obtained composition were filled in spray bottles, provided with a hand-pump, allowing an efficient spraying on the skin.

### Example 6

In the same way as described in Example 1, an aqueous skin tanning composition was composed from:

| | |
|---|---|
| water | 500 g |
| succinic acid | 1.25 g |
| potassium hydroxide | 0.8 g |
| ethyl ester of tyrosine·HCl | 3.0 g |
| d-panthenol | 3.5 g |
| preservative | 1.0 g |
| perfume | 0.5 g |

Spray tins were filled in the usual way with 70 g portions of the obtained composition together with 30 g of dimethoxy methane.

## Claims

1. Use of a composition which is free of fat and/or oil, except in form of a perfume optionally added, and which is composed of at least the following ingredients:
- panthenol in an amount of from 0.1 to 5 % by weight, calculated on the weight of the complete composition,
- tyrosine or a compound from which tyrosine may be formed in situ, in an amount of from 0.1 to 5 % by weight, calculated as tyrosine relative to the weight of the complete composition,
- a pH adjusting, buffering agent to keep the pH of the complete aqueous composition in the range of from 4 to 7, and
- water ad 100 % by weight,
as a skin tanning composition.

2. Use according to claim 1, characterized in that the composition contains d-panthenol in an amount of from 0.1 to 1.0 % by weight, calculated relative to the weight of the complete composition.

3. Use according to claim 2, characterized in that the composition contains d-panthenol in an amount of from 0.4 to 0.8 % by weight, calculated relative to the weight of the complete composition.

4. Use according to any of claims 1 to 3, characterized in that the composition contains a tyrosine ester or phenyl alanine in an amount of from 0.1 to 1.0 % by weight, calculated as tyrosine relative to the weight of the complete composition.

5. Use according to claim 4, characterized in that the composition contains the ethyl ester of tyrosine·HCl or phenyl alanine in an amount of from 0.2 to 0.6 % by weight, calculated as tyrosine relative to the weight of the complete composition.

6. Use according to any of claims 1 to 5, characterized in that the composition contains a pH adjusting, buffering agent to keep the complete aqueous composition in the range of from 4 to 6.

7. Use according to any of claims 1 to 6, characterized in that as pH adjusting, buffering agent the composition includes a combination of lactic acid, succinic acid or citric acid and sodium hydroxide or potassium hydroxide.

8. Use according to any of claims 1 to 7, characterized in that in the composition the pH adjusting, buffering agent is present in an amount of from 0.1 to 1.0 % by weight, calculated relative to the weight of the complete composition.

9. Use according to any of claims 1 to 8, characterized in that in the composition the molar ratio between tyrosine and panthenol is in the range of from 2:1 to 1:4.

10. Use according to claim 9, characterized in that in the composition the molar ratio between tyrosine and panthenol is in the range of from 1:1 to 1:3.

11. Use according to any of claims 1 to 10, characterized in that the composition contains additionally usual carriers and adjuvants to form a milky composition.

12. Use according to any of claims 1 to 10, characterized in that the composition contains additionally usual carriers and adjuvants to form a lotion composition.

13. Use according to any of claims 1 to 11, characterized in that the composition contains a propellant.

14. Use according to claim 13, characterized in that the composition contains carbon dioxide as a propellant in an amount of from 1 to 6 % by weight, calculated relative to the weight of the complete composition.

15. Use according to claim 13, characterized in that the composition contains dimethoxy methane as a propellant in an amount of from 20 to 40 % by weight, calculated relative to the weight of the complete composition.

16. Use according to any of claims 1 to 12, characterized in that an amount of from 2 to 20 ml of the composition is applied per m² of the skin area involved, by means of a hand-pump or a propellant.

17. Skin tanning composition, characterized in that it is composed of the following combination of ingredients:
- panthenol in an amount of from 0.1 to 5 % by weight, calculated on the weight of the complete composition,
- tyrosine or a compound from which tyrosine may be formed in situ, in an amount of from 0.1 to 5 % by weight, calculated as tyrosine relative to the weight of the complete composition,
- a pH adjusting, buffering agent to keep the pH of the complete aqueous composition in the range of from 4 to 7, and
- water ad 100 % by weight.

## Patentansprüche

1. Verwendung einer Zusammensetzung, die frei ist von Fett und/oder Öl, ausgenommen in Form eines gegebenenfalls zugesetzten Duftstoffes, und die aus mindestens den folgenden Bestandteilen zusammengesetzt ist:
- Panthenol in einer Menge von 0,1 bis 5 Gew.-%, berechnet bezüglich des Gewichts der gesamten Zusammensetzung,
- Tyrosin oder eine Verbindung, aus der Tyrosin in situ gebildet werden kann, in einer Menge von 0,1 bis 5 Gew.-%, berechnet als Tyrosin, bezogen auf das Gewicht der gesamten Zusammensetzung,
- ein den pH-Wert einstellendes Puffermittel, um den pH-Wert der gesamten wäßrigen Zusammensetzung im Bereich von 4 bis 7 zu halten, und
- Wasser bis 100 Gew.-%,
als Hautbräunungsmittel.

2. Verwendung nach Anspruch 1, dadurch gekennzeichnet, daß die Zusammensetzung d-Panthenol in einer Menge von 0,1 bis 1,0 Gew.-%, berechnet bezüglich des Gewichts der gesamten Zusammensetzung, enthält.

3. Verwendung nach Anspruch 2, dadurch gekennzeichnet, daß die Zusammensetzung d-Panthenol in einer Menge von 0,4 bis 0,8 Gew.-%, berechnet bezüglich des Gewichts der gesamten Zusammensetzung, enthält.

4. Verwendung nach einem der Ansprüche 1 bis 3, dadurch gekennzeichnet, daß die Zusammensetzung einen Tyrosinester oder Phenylalanin in einer Menge von 0,1 bis 1,0 Gew.-%, berechnet als Tyrosin, in bezug auf das Gewicht der gesamten Zusammensetzung enthält.

5. Verwendung nach Anspruch 4, dadurch gekennzeichnet, daß die Zusammensetzung den Ethylester von Tyrosin · HCl oder Phenylalanin in einer Menge von 0,2 bis 0,6 Gew.-%, berechnet als Tyrosin, in bezug auf das Gewicht der gesamten Zusammensetzung, enthält.

6. Verwendung nach einem der Ansprüche 1 bis 5, dadurch gekennzeichnet, daß die Zusammensetzung ein den pH-Wert einstellendes Puffermittel enthält, um die gesamte wäßrige Zusammensetzung im pH-Bereich von 4 bis 6 zu halten.

7. Verwendung nach einem der Ansprüche 1 bis 6, dadurch gekennzeichnet, daß die Zusammensetzung als das den pH-Wert einstellendes Puffermittel eine Kombination aus Milchsäure, Bernsteinsäure oder Citronensäure und Natriumhydroxid oder Kaliumhydroxid enthält.

8. Verwendung nach einem der Ansprüche 1 bis 7, dadurch gekennzeichnet, daß in der Zusammensetzung das den pH-Wert einstellende Puffermittel in einer Menge von 0,1 bis 1,0 Gew.-%, berechnet bezüglich des Gewichts der gesamten Zusammensetzung, vorliegt.

9. Verwendung nach einem der Ansprüche 1 bis 8, dadurch gekennzeichnet, daß in der Zusammensetzung das Molverhältnis von Tyrosin und Panthenol im Bereich von 2 : 1 bis 1 : 4 liegt.

10. Verwendung nach Anspruch 9, dadurch gekennzeichnet, daß in der Zusammensetzung das Molverhältnis von Tyrosin und Panthenol im Bereich von 1 : 1 bis 1 : 3 liegt.

11. Verwendung nach einem der Ansprüche 1 bis 10, dadurch gekennzeichnet, daß die Zusammensetzung zusätzlich übliche Trägerstoffe und Hilfsstoffe zur Bildung einer milchartigen Zusammensetzung enthält.

12. Verwendung nach einem der Ansprüche 1 bis 10, dadurch gekennzeichnet, daß die Zusammensetzung zusätzlich übliche Trägerstoffe und Hilfsstoffe zur Bildung einer Lotion enthält.

13. Verwendung nach einem der Ansprüche 1 bis 11, dadurch gekennzeichnet, daß die Zusammensetzung ein Treibmittel enthält.

14. Verwendung nach Anspruch 13, dadurch gekennzeichnet, daß die Zusammensetzung Kohlendioxid als Treibmittel in einer Menge von 1 bis 6 Gew.-%, berechnet in bezug auf das Gewicht der gesamten Zusammensetzung, enthält.

15. Verwendung nach Anspruch 13, dadurch gekennzeichnet, daß die Zusammensetzung Dimethoxymethan als Treibmittel in einer Menge von 20 bis 40 Gew.-%, berechnet in bezug auf das Gewicht der gesamten Zusammensetzung, enthält.

16. Verwendung nach einem der Ansprüche 1 bis 12, dadurch gekennzeichnet, daß eine Menge von 2 bis 20 ml der Zusammensetzung pro m² der herangezogenen Hautfläche mittels einer Handpumpe oder eines Treibmittels angewandt wird.

17. Hautbräunungsmittel, dadurch gekennzeichnet, daß es aus der folgenden Kombination von Bestandteilen zusammengesetzt ist:
- Panthenol in einer Menge von 0,1 bis 5 Gew.-%, berechnet bezüglich des Gewichts der gesamten Zusammensetzung,
- Tyrosin oder eine Verbindung, aus der Tyrosin in situ gebildet werden kann, in einer Menge von 0,1 bis 5 Gew.-%, berechnet als Tyrosin, in bezug auf das Gewicht der gesamten Zusammensetzung,
- ein den pH-Wert einstellendes Puffermittel, um den pH-Wert der gesamten wäßrigen Zusammensetzung im Bereich von 4 bis 7 zu halten, und
- Wasser bis zu 100 Gew.-%.

## Revendications

1. Utilisation d'une composition qui est exempte de graisse et/ou d'huile, sauf sous la forme d'un parfum optionnellement ajouté, et qui est constituée d'au moins les ingrédients suivants :
- du panthénol à raison de 0,1 à 5 % en poids, calculés sur le poids de la composition totale,
- de la tyrosine ou un composé à partir duquel de la tyrosine puisse être formée in situ, à raison de 0,1 à 5 % en poids, calculés sous forme de tyrosine en fonction du poids de la composition totale,
- un agent tampon régulateur de pH pour maintenir le pH de la composition aqueuse totale dans une plage de 4 à 7, et
- de l'eau à 100 % en poids,
comme composition pour le bronzage de la peau.

2. Utilisation selon la revendication 1, caractérisée en ce que le composition contient du d-panthénol à raison de 0,1 à 1,0 % en poids, calculés en fonction du poids de la composition totale.

3. Utilisation selon la revendication 2, caractérisée en ce que la composition contient du d-panthénol à raison de 0,4 à 0,8 % en poids, calculés en fonction du poids de la composition totale.

4. Utilisation selon l'une ou l'autre des revendications 1 à 3, caractérisée en ce que la composition contient un ester de tyrosine ou de la phényl alanine à raison de 0,1 à 1,0 % en poids, calculés sous forme de tyrosine en fonction du poids de la composition totale.

5. Utilisation selon la revendication 4, caractérisée en ce que la composition contient l'ester éthylique de tyrosine.ClH ou de phényl alamine à raison de 0,2 à 0,6 % en poids, calculés sous forme de tyrosine en fonction du poids de la composition totale.

6. Utilisation selon l'une ou l'autre des revendications 1 à 5, caractérisée en ce que la composition contient un agent tampon régulateur de pH pour maintenir la composition aqueuse totale dans une plage de 4 à 6.

7. Utilisation selon l'une ou l'autre des revendications 1 à 6, caractérisée en ce que comme agent tampon régulateur de pH la composition contient une combinaison d'acide lactique, d'acide succinique ou d'acide citrique et d'hydroxyde de sodium ou d'hydroxyde de potassium.

8. Utilisation selon l'une ou l'autre des revendications 1 à 7, caractérisée en ce que dans la composition l'agent tampon régulateur de pH est présent à raison de 0,1 à 1,0 % en poids, calculés en fonction du poids de la composition totale.

9. Utilisation selon l'une ou l'autre des revendications 1 à 8, caractérisée en ce que dans la composition le rapport molaire entre la tyrosine et le panthénol est compris entre 2 à 1 et 1 à 4.

10. Utilisation selon la revendication 9, caractérisée en ce que dans la composition le rapport molaire entre la tyrosine et le panthénol est compris entre 1 à 1 et 1 à 3.

11. Utilisation selon l'une ou l'autre des revendications 1 à 10, caractérisée en ce que la composition contient les véhicules et adjuvants habituels pour former une composition laiteuse.

12. Utilisation selon l'une ou l'autre des revendications 1 à 10, caractérisée en ce que la composition contient les véhicules et adjuvants habituels pour former une composition de lotion.

13. Utilisation selon l'une ou l'autre des revendications 1 à 11, caractérisée en ce que la compoition contient un agent de propulsion.

14. Utilisation selon la revendication 13, caractérisée en ce que la composition contient de l'anhydride carbonique comme agent de propulsion à raison d'1 à 6 % en poids, calculés en fonction du poids de la composition totale.

15. Utilisation selon la revendication 13, caractérisée en ce que la composition contient du di-méthoxy-méthane comme agent de propulsion à raison de 20 à 40 % en poids, calculés en fonction du poids de la composition totale.

16. Utilisation selon l'une ou l'autre des revendications 1 à 12, caractérisée en ce qu'une quantité de 2 à 20 ml de la composition est appliquée par mètre carré de la surface de peau concernée, au moyen d'une pompe à main ou d'un agent de propulsion.

17. Composition pour le bronzage de la peau, caractérisée en ce qu'elle est constituée de la combinaison d'ingrédients suivante :
- du panthénol à raison de 0,1 à 5 % en poids, calculés sur le poids de la composition totale,
- de la tyrosine ou un composé à partir duquel de la tyrosine peut être formée in situ, à raison de 0,1 à 5 % en poids, calculés sous forme de tyrosine en fonction du poids de la composition totale,
- un agent tampon régulateur de pH pour maintenir le pH de la composition aqueuse totale dans une plage de 4 à 7, et
- de l'eau à 100 % en poids.
